# EUROPEAN PATENT APPLICATION

(11) **EP 0 613 672 A1**
(43) Date of publication of application: **07.09.1994**
(21) Application number: 94103053.8
(22) Date of filing: 01.03.1994
(51) Int. Cl.: A61F 13/26

(54) **Water-soluble tampon applicator**

(30) Priority: 01.03.1993 US 24614
(71) Applicant: TAMBRANDS, INC., White Plains, New York 10604 (US)
(72) Inventor: Albright, Mark D., Port Solent, Portsmouth PO6 4SY (GB); Tarr, Warren, Turners Falls, Mass. 01376 (US)
(74) Representative: Hansen, Bernd, Dr. Dipl.-Chem.

(57) **Abstract**

A dissolvable tubular tampon applicator made from a water-soluble polymer or substrate having a surface (13,14) coated with a degradable, water-insoluble polymeric material (15) and methods for making the same. The water-insoluble polymeric material can be wax, hydrogenated vegetable oil, or food grade shellac. The water-soluble polymer can be thermoplastic starch, polyvinyl alcohol, poly(alkylene oxide), hydroxyethyl cellulose, hydroxypropyl cellulose, polyvinyl pyrrolidone, polyacrylamide, polyacrylic acid or polyvinyl methyl ether-co maleic anhydride. The method to make the article can involve tumble-coating the exterior surface of the water-soluble polymeric tubular applicator with the heat-softened water-insoluble polymeric material.

## Description

### FIELD OF THE INVENTION

This invention relates to a water-soluble disposable article such as a catamenial tampon applicator and methods for producing the same. More particularly, this invention relates to an applicator comprising at least one elongated tubular member comprised of a water-soluble polymer coated on the exterior surface thereof with one or more water-insoluble polymeric material(s) and, methods for producing the same.

### BACKGROUND OF THE INVENTION

The standard construction for a tampon applicator is a pair of telescoping tubes, the outer tube carrying the tampon formed of a compressed absorbent material (sometimes referred to as a "pledget", particularly if formed of a uncompressed super-absorbent material) and the inner tube serving as a plunger for ejecting the tampon. Tampon applicators in current commercial use are typically made from either an insoluble plastic or a flushable smooth surfaced cardboard. Plastic tampon applicators are preferred by a certain number of women since they are perceived to be smoother and include petal-shaped lobes covering the forward end of the tampon which aids in the insertion of the device and in retaining and protecting the tampon while it is in the outer tube. Plastic tampon applicators are typically made from water insoluble polyethylene using an injection-molding process. These plastic applicators have always posed a significant environmental and aesthetic problem, in spite of their commercial success. They should never be disposed of by flushing, yet once soiled, women reluctantly are otherwise forced to dispose of them with the dry trash.

For this reason flushable cardboard applicators have retained their popularity while those in this art have continued to search for over half a century for an applicator that would combine the benefits of both types of applicators. All of the currently used commercial plastic applicators remain ill-suited for toilet disposal. While such solid insoluble applicators can be forced to flush, they settle in septic tanks without decomposing. Moreover, as is also already well recognized in the prior art, they accumulate on the screens in waste-water treatment plants, creating blockages. If the screens do not stop them, they escape into environment intact, often washing up on beaches. Because plastic tampon applicators typically neither float on the surface nor settle to the bottom of setting tanks, they are not removed as sludge or by skimmers. In view of such environmental and aesthetic considerations, it is thus highly desirable that the plastic applicator be rapidly water-dispersible (dissolvable) and biodegradable, but only after use. Such properties have not yet been achieved commercially, in spite of efforts to achieve these properties for a plastic based material which have been on-going for at least the past forty years (see patent 2,518,486).

Herring, U.S. Patent No. 5,002,526 relates to using a modified, self plasticizing polyvinyl alcohol as a material for a tampon applicator. This patent gives a good review of the problems of the prior art and of long-sought goals for developing a commercially-viable disposable catamenial applicator (including listing desirable features). Unfortunately, Herring's own applicator did not adequately solve these problems. It can take up to two hours to dissolve. This can cause embarrassment when the applicator does not clear the toilet when flushed, as can too often happen. Even when successfully flushed, Herring's applicator can still be a problem for waste systems. For instance, Herring's applicator can survive intact long enough to get caught in plumbing, e.g. constricted pipes or traps within a building, and initial blockages prior to the applicator dissolving, thereby causing or exacerbating clogging of pipes.

The use of polyvinyl alcohol ("PVOH") as a material for a device for dispensing pharmaceutical preparations into a body cavity can be found suggested as far back as in Mende, U.S. Patent No. 2,518,486 and Bentz, U.S. Patent No. 2,516,846. However, Hanke, U.S. Patent No. 3,882,869 correctly points out that Mende's approach is unworkable in a commercial environment because the PVOH becomes unstable in the presence of moisture laden air, becoming prematurely sticky (or even slimy), a problem that has plagued use of PVOH in tampon applicators to this day (in spite of numerous schemes to overcome this problem without losing effective disposability). Hanke also stated that PVOH is costly to fabricate, since it must be cast from solution and then molded to shape (see Hanke at col. 1, lines 49-59). As an alternative to Mende's approach, Hanke suggests using a polyethylene oxide polymer or hydroxypropyl cellulose containing between about 50 and 75 percent by weight of a filler as talc. Other approaches to tampon applicators may be found in U.S. Patent Nos. 3,724,462, 3,882,196, 3,911,917, 3,954,104, 4,099,976, and 4,372,311.

Potts, U.S. Patent No. 4,372,311 is directed to disposable articles, including water soluble applicator tubes for tampons, made from PVOH or other water-soluble polymers which are coated with a degradable water-insoluble polymeric film or laminate selected from one or more of a cyclic ester polymer, a poly(β-hydroxy butyrate), a dialkanoyl polymer such as polyesters and polyurethanes derived from aliphatic polyols, or an ethylene polymer. The coating is applied by solvent system coating techniques (e.g. dip, spray, brush, etc.) to a thickness of 0.5 to 5.0 mils. In the examples, upon dissolving the article typically left a slowly degradable water-insoluble thin film.

There are several disadvantages to Potts' approach. His coating materials are relatively expensive. Solvent system coating techniques require a solvent carrier; and, for Potts' coating materials, the solvent must be at an elevated temperature such as 200° to 300°F. The severe temperature of the coating, when applied to the applicator, can cause marring of the applicator surface or distortion of the applicator.

Other patents of interest include Gross, U.S. Patent No. 2,223,611 and Pohl, U.S. Patent No. 2,474,188. Gross relates to a powder dispensing paper tube for insertion into a body cavity which is coated with a water proofing compound such as paraffin. Pohl is directed to a disposable enema tube which may be made of cheap molded wood-pulp or other materials and is provided with a coating of water-proofing material. These patents suffer from the problem that the underlying material e.g., paper or wood pulp, are not water-soluble (though capable of slow disintegration and ultimate biodegradability).

A basic problem of uncoated water-soluble polymer applicators such as applicators from PVOH or poly(alkylene oxide), e.g., poly(ethylene oxide) polymers, is that they tend to become tacky in high humidity or upon contact with moisture such that the applicator interferes with the insertion of the tampon by jamming of the closely interfitting tubes or even worse by adhering to sensitive vaginal tissue. Additionally, the often slimy feel is very unesthetic. Indeed, this is a basic problem attendant to the water-soluble nature of the polymer, e.g., premature solubilization of the applicator or the surface thereof. Likewise, since the tampon and applicator therefor can contact moisture from hands or fingers during use and are being inserted into a naturally moist environment, the applicator can become quickly tacky during use, thus interfering with withdrawal of the applicator and proper placement of the tampon. These problems not only mean reduced shelf-life for the tampon, but also, discomfort for women, as well as possible leakage from an ineffectively inserted tampon (thereby causing embarrassment and mess for the user).

Thus, it would be desirable to have a water-soluble polymeric tampon applicator which effectively resists tackiness from moisture prior to and during use, yet which dissolves readily and rapidly, and which is less expensive and easier to produce than previously proposed applicators. It is desirable to have an improved coated water-soluble polymeric tampon applicator that resists tackiness from ambient moisture prior to use, but rapidly dissolves upon total immersion after use. It is further desired to be able to obtain such a coated applicator by a simple inexpensive method, e.g. without spray-coating.

U.S. Patents Nos. 533,303, 2,560,820, 3,438,797, 3,798,338 and 3,911,860 relate to tumble coating techniques, but with no suggestion of relevance to the catamenial tampon applicator art. For instance, Lee, U.S. Patent No. 533,303 is directed to coating a catheter tube with varnish by a tumbling operation. In particular, none of these patents propose or even suggest tumble-coating a water-soluble polymeric tampon applicator with a water-insoluble polymeric material.

From the foregoing, heretofore a tampon applicator comprising at least one elongated tubular member comprising a water-soluble polymer coated on an exterior surface thereof with one or more water-insoluble polymeric materials selected from wax, hydrogenated vegetable oil, and food grade shellac, and the like has not been taught or suggested. Likewise, heretofore a method for producing a water-resistant, degradable tampon applicator comprising at least one elongated tubular member comprising forming the tubular member from the water-soluble polymer and coating an exterior surface of the tubular member with a water-insoluble polymeric material by tumbling the tubular member in the presence of the water-insoluble polymeric material at a temperature above the melting point of the water-insoluble polymeric material and below the melting or distorting temperature of the water-soluble polymer, has not been taught or suggested.

### OBJECTS AND SUMMARY OF THE INVENTION

It is an object of the invention to provide a commercially effective and aesthetically-acceptable rapidly-disintegrating water-soluble disposable tampon applicator with good shelf life.

It is an object of the invention to provide a water-soluble tampon applicator which rapidly dissolves when immersed in water after use yet effectively resists becoming tacky from ambient moisture before and during use.

It is further object of the invention to provide a simple and inexpensive method for producing tampon applicators of the invention.

Thus, the present invention provides a tampon applicator comprising at least one elongated tubular member comprising a water-soluble polymer coated on at least the exterior surface thereof with one or more water-insoluble polymeric material selected from the group consisting of wax, hydrogenated vegetable oil, and food grade shellac.

The present invention also provides a method for producing a water-resistant, degradable tampon applicator comprising at least one elongated tubular member comprising forming said tubular member from a water-soluble polymer and coating on at least an exterior surface of the tubular member with a water-insoluble polymeric material by tumbling the tubular member in the presence of the water-insoluble polymeric material at a temperature above the melting point of the water-insoluble polymeric material and below the melting temperature of the water-soluble polymer.

Further objects and embodiments will be apparent from the following detailed description.

### BRIEF DESCRIPTION OF THE DRAWINGS

Fig. 1 shows an applicator of the invention; and
Figs. 2 and 3 each show a cross-section along lines 2-2 and 3-3, respectively, of the applicator of Fig. 1.

### DETAILED DESCRIPTION

Any reasonably water-soluble polymer capable of being formed into a functioning applicator may be used to fabricate the tampon applicator of the invention. Water-soluble polymers which are suitable for the tampon applicator of the invention include poly(alkylene oxide) (e.g., poly(ethylene oxide)), hydroxyethyl cellulose, hydroxypropyl cellulose, polyvinyl alcohol, polyvinyl pyrrolidone, polyacrylamide, polyacrylic acid, poly(vinyl methyl ether-co maleic anhydride), and, thermoplastic starch. As to polyvinyl alcohol, "VINEX" (not modified as in U.S. Patent No. 5,002,526) is commercially available. With respect to thermoplastic starch "NOVON" is commercially available. With regard to poly(alkylene oxide) e.g., poly(ethylene oxide) polymers, Potts, U.S. Patent No. 4,372,311, incorporated herein by reference, cites, inter alia, U.S. Patents Nos. 3,417,064 and 3,763,277. Presently, Vinex copolymer is preferred as the water-soluble polymer for the tampon applicator of the invention, e.g., VINEX 2025 referred to by AIR PRODUCTS as AP# HTS-89-0922.

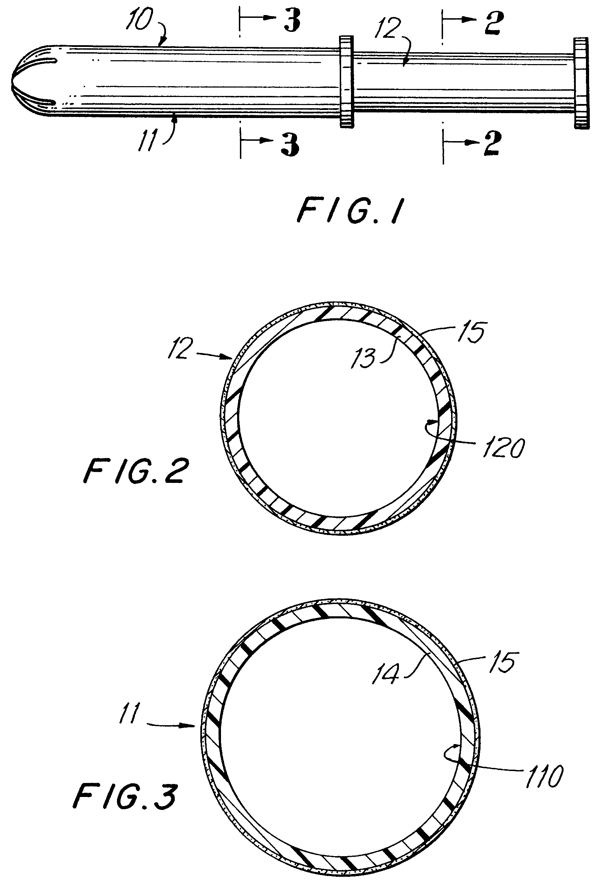

As mentioned earlier, a tampon applicator (10; Fig. 1) typically comprises at least one tubular member but most typically comprises a pair of telescoping tubes (11, 12; Fig. 1). The tube or tubes can be formed from the water-soluble polymeric material by any suitable means such as injection molding, extrusion, or both injection molding and extrusion (for instance, the outer tube can be made by injection molding and the inner tube by extrusion). Herring, U.S. Patent No. 5,002,526, col. 5 lines 44-52, cites Rubin, "Injection Molding" in Herman F. Mark et al., eds. "Encyclopedia of Polymer Science and Engineering," Vol. 8, John Wiley & Sons, New York, 1987, pp 102-138 for a general discussion of injection molding and, Kruder, "Extrusion" in Herman F. Mark et al., eds., "Encyclopedia of Polymer Science and Engineering," Vol. 6, John Wiley & Sons, New York, 1986, pp. 571-631 for a general discussion of extrusion. U.S. Patent No. 5,002,526 is hereby incorporated herein by reference.

After the tampon applicator is formed from the water-soluble polymeric material, it is coated with a water-insoluble polymer. The water-insoluble polymer may be applied preferably by a tumbling coating method described below, or by other known more expensive or less effective methods such as spray coating, brushing, dip coating, extrusion, co-extrusion and the like. The water-insoluble polymeric material is preferably applied to the water-soluble polymer at a temperature below the melting point of the water-soluble polymer, more preferably below the softening point or distortion temperature of the water-soluble polymer. As discussed below, preferably the applicator is preformed from the water-soluble polymer and then coated with the water-insoluble polymeric material. However, by judicious matching of melting points and compatible flow properties the water-soluble portion and the coating thereon could possibly be made simultaneously or nearly simultaneously for instance, by extrusion or co-extrusion.

For applying certain water-insoluble polymers to the water-soluble portion of the tampon applicator to make the tampon applicator of the invention, tumble coating is preferred.

In a simple embodiment of the tumble coating method of the invention, an apparatus which has a vessel to apply sufficient tumbling and heat is employed. In a preferred embodiment, initially a sufficient quantity of the water-insoluble polymer, for instance in pellet form, is added to the vessel. The vessel tumbles and is maintained at a suitable temperature above the melting point of the water-insoluble polymer; for example 10° to 20°F above the melting point. Thus, preferably the inner surface of the vessel is coated with the water-insoluble polymer. Then, the preformed applicators (of the water-soluble polymer) are introduced into the vessel. The vessel tumbles and is maintained at a suitable temperature above the melting point of the water-insoluble polymer and below the melting point or softening point or distortion temperature of the water-soluble polymer (for instance, 10° to 20°F above the melting point of the water-insoluble polymer, and below the melting point or more preferably below the softening point or distorting temperature of the water-soluble polymer). The water-soluble applicator is thus "painted" on at least the exterior surface thereof with a coating of water-insoluble polymer (some water-insoluble polymer may thinly coat a small portion of the inner surface of the tube or cylinder comprising the preformed applicator). Of course, one need not initially coat the inner surface of the vessel. The preformed applicators of the water-soluble polymer and the water-insoluble polymer, e.g., in pellet form, can be introduced into the vessel simultaneously or nearly simultaneously and, the vessel can apply tumbling and heat so that the water-soluble applicators are coated with the water-insoluble polymer.

A typical clothes dryer can be employed as a tumble coater. It is preferred to also use an inert coating agent such as glass beads, plastic pellets, ball bearings and the like which act to evenly distribute the melted water-insoluble polymer, e.g., wax, for instance, on the surface of the applicators. Other apparatus which can be used in the practice of the invention include any commercially available tumble coaters (e.g., the type used to coat pharmaceutical tablets), and, fluid bed vibrating equipment and vibrating pans (e.g., the type used in the confectionary industry for coating candies). When the water-insoluble polymer is a wax, e.g., paraffin or ozokerite or food grade wax, the melting point is in the range of about 120° to 145°F and the vessel (dryer drum) can be 10° to 20°F above that range. It is preferred that the temperature used while coating be below or not exceed about 170°F because above this temperature the PVOH (VINEX) in preferred applicators can become distorted.

In addition to wax such as soft paraffin, carnauba, ozokerite, food grade and petroleum based wax, hydrogenated vegetable oil (commercially available as "STEROTEX") or food grade shellac are preferred water-insoluble polymeric materials for use in making the tampon applicators of the invention.

With respect to wax as the water-insoluble polymeric material, the wax can be a natural wax such as an animal wax (e.g., beeswax, spermaceti, lanolin, shellac wax), vegetable wax (e.g., carnauba, candelilla, bayberry, sugar cane) or mineral wax (e.g., fossil or earth wax such as ozokerite, ceresin, montan, or petroleum wax such paraffin, microcrystalline, petrolatum, or slack or scale wax) or, synthetic wax, chlorinated naphthalenes (e.g., "Halowax") and hydrocarbon type wax, for instance via Fischer-Tropsch synthesis. In general, waxes are thermoplastic, though not considered in the family of plastics, and have common properties such as smooth texture, nontoxicity, freedom from objectionable odor and color, good dielectric properties, insoluble in water, and soluble in most organic solvents. Indeed, a distinction between wax such as synthetic wax for use in this invention and ethylene polymers proposed heretofore is that wax is not a plastic and is thus different from previously proposed ethylene polymers (such as in Potts, U.S. Patent No. 4,372,311).

When the water-insoluble polymer is a food grade shellac, such as those available from Mantrose-Hauser, it is preferred to apply it to the preformed water-soluble applicator by spray-coating with an alkyl alcohol, e.g., isopropyl alcohol, as the carrier. This spray-coating, like the tumble-coating, is preferably below the melting point, more preferably below the softening point or distorting temperature of the water-soluble polymer.

The coating of the water-insoluble polymer (15; Figs. 2, 3) is preferably 0.1 to 1.0 mils, more preferably 0.5 mils in thickness. The coating (15; Figs. 2, 3) is preferably on the outer exterior surface (13, Fig. 2; 14, Fig. 3) so as to allow the interior (inner) surface (120, Fig. 2; 110, Fig. 3) of the tube (11, 12; Figs. 1, 2, 3) or at least some reasonably effective portion thereof to contact the water of a toilet bowl so as to facilitate the speed of dissolving. The outer coating is preferably 0.5 mils thick so as to provide an adequate, uniform coating which substantially does not delaminate or become substantially porous due to cracking or peeling.

The coating can, of course, be of any suitable thickness, depending on the particular water-insoluble polymer. However, too thin a layer will not provide sufficient or uniform coating and, too thick a layer will be too costly, an increased burden on the environment, and subject to delamination. Likewise, other water-insoluble polymeric materials, for instance, preferably of a same or similar nature as those above-mentioned, may also be employed in the practice of the invention. For instance, hydrophobic lubricants such as mineral oil, silicone gel or fluid, petroleum jelly and the like can also be used in the practice of the invention. However, in selecting a water-insoluble polymeric material, it is preferred to avoid those which significantly and/or deleteriously migrate into the water-soluble polymer and/or are or become tacky *in situ*.

The practice of the invention, especially the wax and vegetable oil embodiments present advantages. These advantages include no need for application of the coating by way of a solvent system, ability to apply the coating at a lower temperature, without marring the water-soluble polymer and, lower cost to produce, not only because the preferred technique (tumble-coating) is simpler, but also because the preferred coating materials are less expensive than those previously used. Further, as discussed in the Examples below, the present invention provides an applicator which has a good shelf life yet can dissolve in 12 to 20 minutes, generally about 15 (in some instances about 12 to about 30 minutes, generally about 20 minutes; but in all instances at least softening or loss of rigidity occurs in about 10 to about 15 minutes). Thus, the present invention can provide a tampon applicator which does not present any substantial environmental or clogging problem or threat thereof to plumbing, septic tanks, waste water treatment plants and the like. Indeed, in even clogged or constricted pipes or traps the applicator of the invention can dissolve.

Moreover, the present invention provides a tampon applicator which, in terms of preference by women when used to insert a tampon, is not only surprisingly as good as comparable commercially available non-dissolvable plastic applicators (see Example 4), a highly desirable result, but is even found by a significant small number of the women to be even better. While the margin of preference by women for the present invention over the commercially available plastic applicator may not be overall statistically significant, the fact that women do not seem to notice any distinct difference between the present invention and the commercially available non-dissolvable plastic applicator shows that the present invention is eminently feasible commercially as a replacement for the currently available polyethylene non-dissolvable plastic applicator (see Example 4). This is surprising and unexpected because a significant number of the consumer test panelists rejected the uncoated Vinex applicator in favor of the standard, commercially available polyethylene applicator (see Example 3). Furthermore, the fact that women do not seem to notice any distinct difference in inserting a tampon between the present invention and the commercially available non-dissolvable plastic (polyethylene) applicator (Example 4) may also be a true test of the utility of the present invention because merely incorporating a tampon in an applicator does not show that the applicator can contact sensitive vaginal tissue, suitably insert a tampon into a woman's body, and be accepted by women for this purpose.

However, while this description has focused upon the invention being embodied in a tampon applicator and a method for producing the same, the present invention also comprehends non-catamenial devices capable

of utilizing disposable applicator tubes. Preferably the coated water-soluble applicator loaded with a tampon is packaged in a moisture-proof envelope to assure good shelf-life prior to actual use. Since such an envelope can be non-descript in appearance and not subject to soiling, its disposal with dry waste is not a significant problem, especially if made from biogradable materials.

The following examples are given by way of illustration only and are not to be considered as limitations of the present invention as many apparent variations of which are possible without departing from the spirit or scope thereof.

### EXAMPLES

### EXAMPLE 1 - PREPARATION OF COATED AND UNCOATED APPLICATORS

A batch of identical typical telescoping tubular tampon applicators were formed of polyvinyl alcohol (Vinex) by injection molding. These were made in molds having a shape and dimensions typical for a commonly available commercial polyethylene tampon applicator; namely, Petal Soft. More specifically, the wall thickness of the outer tube ranged from .03 cm to .08 cm¹. These applicators are "uncoated" PVOH applicators. Paraffin wax was introduced into a modified clothes dryer, and the drum set in motion with heat supplied to maintain a temperature about 10 to 20°F above the melting point of the wax; in this instance, the dryer temperature was about 94°C. After the drum was coated with the wax, some of the uncoated PVOH applicators were added and tumbled at this temperature until coated (after about 5 minutes in dryer). The exterior coating was smooth, unbroken and about 0.0005 inches thick. The same procedure was employed with ozokerite and Stereotex wax with similar good results.
¹ For purposes of these tests the inner (ejector) tube was made of cardboard.

### EXAMPLE 2 - DISSOLVING APPLICATORS

The still uncoated PVOH applicators remaining from Example 1 were each placed in a beaker with about 450 ml tap water at 30°C. By eleven minutes it became visually apparent that each applicator was dissolving. By fourteen minutes each applicator had mostly dissolved. By seventeen minutes each applicator was completely disintegrated and substantially dissolved. This procedure and observation was repeated using PVOH applicators from the same batch, but coated with wax as prepared in Example 1 with similar results, except that small wax globules were observed remaining. In particular, the amount of dissolution was checked every five minutes and recorded every fifteen minutes, unless anything unusual occurred. The applicator of the invention was observed to dissolve in some instances in about 12 to about 20 minutes, generally about 15 minutes. In all instances, complete dissolution occurred by or during the 25 minute interval. Thus, in some instances it took about 12 to about 30 minutes, generally about 20 minutes, for the inventive applicators to dissolve; but, in this instance, softening or loss of rigidity occurred in about 10 to about 15 minutes.

Accordingly, in all instances, by about 10 to about 15 minutes at least softening or loss of rigidity occurred in the inventive applicators. A further test was run with uncoated, ozokerite-coated and Sterotex-coated PVOH applicators, prepared as per Example 1. These three types of applicators were each immersed in a beaker with 200 ml tap water at 30°C. Each had dissolved in 25 minutes with no noticeable difference in the rate of dissolution. Thus, the wax coating of the inventive applicators does not seem to significantly affect the rate of dissolution relative to the uncoated applicator. Since small wax globules (particulate to microparticulate in nature) remain dispersed in the water upon dissolution of the applicator, biodegradable and/or natural and/or food quality water-insoluble polymeric materials are preferred for use in the invention. Moreover, since the applicator is of a typical commercial size, it can clear the toilet in a flush and, since it rapidly softens and can dissolve in about 12 to 20 (generally about 15 minutes) the applicator of the invention does not present any substantial environmental or clogging problem or threat to plumbing, septic tanks, or waste-water treatment plants. Furthermore, the speed at which the inventive applicator softens or loses stiffness or rigidity is significant because the consumer can visually confirm that the applicator is performing as intended, i.e., dissolving (thereby providing confidence that she is not leaving the applicator behind for those subsequently using the facilities to see, and, that it will clear the toilet in a flush).

### EXAMPLE 3 - COMPARISON TEST OF UNCOATED WATER-SOLUBLE APPLICATOR AND COMMERCIALLY AVAILABLE, NON-DISSOLVABLE PLASTIC APPLICATOR

The uncoated PVOH applicator of Example 1, having the same dimensions and form as the applicator for the commercially available Tambrands product known as the Petal Soft Super Tampon, was loaded with the standard tampon for that type of applicator. A panel of fifty Super Tampon users was recruited. Each panelist was given ten samples of the Super Tampon with the uncoated Petal PVOH applicator ("S") and ten samples of the standard commercial Petal Soft Super Tampon with the normal polyethylene applicator ("R"). The panelists were instructed to use these products alternately during their next menstrual cycle. The products were blind wrapped and the order of presentation was balanced. Thirty-five of the panelists completed the experiment and responded to questions regarding which tampon (S or R) was preferred. In order to assure no bias from the content of the questions, several questions were directed to matters not of any significance to the areas of actual interest. Also voluntary comments were solicited with respect to each question. The results are set forth below.

**TABLE I**

| **OVERALL PREFERENCE** | |
|---|---|
| Tampon | Number of Panelists |
| R | 23* |
| S | 4 |
| No Preference | 8 |

| | |
|---|---|
| * = Significant >90% level of confidence | |

Reasons given for preferring R were as follows:

**TABLE II**

| **REASONS FOR PREFERENCE FOR R (BASE = 23)** | |
|---|---|
| REASON | NUMBER OF PANELISTS |
| Easy to insert/applicator easier to use | 8 |
| Effective, reliable, serves the purpose | 2 |
| Applicator not sticky | 2 |
| Easy to use/convenient | 2 |
| Absorbency/protection | 1 |
| Good/more absorbent-very absorbent | 1 |
| Plastic applicator made insertion easier/comfortable | 1 |
| Easier to expel tampon from applicator | 1 |
| Comfort | 1 |
| Comfort while wearing | 1 |
| All other comments on withdrawal/removal | 1 |
| Physical characteristics of applicator | 1 |
| Plastic applicator | 1 |
| All other comments on package/wrapper | 1 |
| Miscellaneous | 2 |

**TABLE III**

| **SPECIFIC PREFERENCES** | | | | |
|---|---|---|---|---|
| | Number of Panelists for: | | | |
| SPECIFIC PREFERENCE | TAMPON R | TAMPON S | NO PREFERENCE | NO ANSWER |
| Protection from leakage | 8 | 3 | 24 | 0 |
| Ease of applicator insertion | 20* | 2 | 12 | 1 |
| Ease of pushing tampon out of applicator | 18* | 1 | 16 | 0 |
| Ease of applicator withdrawal | 14* | 2 | 19 | 0 |
| Ease of tampon withdrawal | 7 | 2 | 26 | 0 |

| | | | | |
|---|---|---|---|---|
| * = Significant > 90% level of confidence | | | | |

As to the preference for protection from leakage, one panelist viewed "S" as "terrible", another panelist stated that "S" leaked on a light flow day and yet two other panelists respectively stated that "R" was good for heavy flow days and that "R" did not leak. With respect ease of insertion, panelist comments included substantially the following:
S at times were painful.
S seemed rougher.
S Applicator had a white residue and made it more difficult to insert.
R had easy slide.
R was smooth, made insertion easier.
Did not like applicator S.
R was smoother, easier withdrawal.
S had some kind of coating.
S had trouble with pushing applicator in; never seemed comfortable.
R was easier.
No problem from inserting R.
R seemed easier to insert.
Plastic applicator of R was easier to insert.

As to ease of pushing the tampon out of the applicator, panelist comments included that R seemed easier to use, was smoother, and did not cause discomfort whereas S: "seemed rougher"; "was so painful"; was "very hard"; provided "little control of direction"; "was difficult [to use] and often pinched the side of the skin"; and was "trouble" to use.

With respect to ease of applicator withdrawal, the panelist comments included:
S seemed to resist withdrawal.
S was hard to grasp - had to hold tight and pull hard.
S seemed to come apart.
R was smooth.
S was nasty - R much easier.
S pinched some when withdrawn.
S was very difficult to withdraw.
No stickiness with R.

The panelists rated the overall performance of tampons R and S on a five point scale (5 = excellent, 1 = poor) as set forth in Table IV.

**TABLE IV**

| **OVERALL PERFORMANCE** | | |
|---|---|---|
| PERFORMANCE | TAMPON R | TAMPON S |
| Excellent | 6 | 0 |
| Very good | 15 | 7 |
| Good | 6 | 5 |
| Fair | 4 | 9 |
| Poor | 3 | 11 |
| No Answer | 1 | 3 |
| **Mean** | **3.50******* | **2.25** |

| | | |
|---|---|---|
| * = Significant >90% level of confidence. | | |

Tampon S was disliked for reasons shown in Table V.

**TABLE V**

| **DISLIKES OF TAMPONS (BASE=30)** | |
|---|---|
| REASON | NUMBER OF PANELISTS |
| Difficult to insert | 9 |
| Uncomfortable/painful insertion | 5 |
| Difficult to expel tampon from applicator | 5 |
| Difficult to remove applicator | 4 |
| Uncomfortable | 3 |
| Physical characteristics of applicator/the applicator | 3 |
| Absorbency/protection | 3 |
| All other comments in insertion | 2 |
| Difficult to withdraw/remove | 2 |
| White residue | 2 |
| Deordorancy/fragrance | 2 |
| More leakage | 1 |
| Not good for heavy days - need pad/shield on heavy days | 1 |
| Plastic applicator difficult to insert (pinching, rough) | 1 |
| Uncomfortable to wear | 1 |
| Uncomfortable/painful removal/withdrawal | 1 |
| Too large/too long | 1 |
| Material comes apart/shredded | 1 |
| Applicator came apart | 1 |
| Sticky-hard to hold | 1 |
| Disposability | 1 |
| Not flushable/not biodegradable | 1 |
| Tube absorbs menses | 1 |
| All comments on wrapper packaging | 1 |
| Miscellaneous | 1 |

As shown by the above results, the panelists did not know which applicator was made from PVOH and which was made from polyethylene (note "dislike": "Not flushable/not biodegradable", Table V). Moreover, the above results show that the great majority (23/35) preferred the tampon with the polyethylene applicator (R). Tampon R was also significantly preferred for "ease of applicator insertion"_{,} "ease of pushing tampon from applicator" and "ease of applicator withdrawal". Tampon R had a statistically significant mean overall performance rating of 3.50. Further, a significantly larger proportion of the panelists had favorable comments regarding Tampon R than regarding Tampon S, and similarly, a significantly larger proportion of the panelists had unfavorable comments relating to Tampon S than to Tampon R. The unfavorable comments associated with insertion and the physical characteristics of the applicator were especially frequent for Tampon S.

### EXAMPLE 4 - COMPARISON TEST OF WAX COATED WATER-SOLUBLE APPLICATOR AND COMMERCIALLY AVAILABLE, NON-DISSOLVABLE PLASTIC APPLICATOR

The ozokerite wax coated PVOH applicator ("M") of Example 1, having the form of a Petal Soft Super tampon applicator, was loaded with the same type of tampon as a commercially available Petal Soft Super tampon. In other words, applicator "M" is identical to applicator "S" in Example 3, except for the added wax coating. A panel of fifty Super Tampon users was recruited. Each subject received ten samples of the Super Petal Soft tampon with the wax coated PVOH applicator ("M") and ten samples of the standard commercial Super Petal Soft Tampon with the polyethylene applicator ("R"). The panelists were instructed to use these products alternatively during their next menstrual cycle. The products were blind wrapped and the order of presentation was balanced. Thirty-three of the panelists completed the experiment and responded to questions regarding which tampon (M or S) was preferred. The results are set forth below.

**TABLE VI**

| **OVERALL PREFERENCE** | |
|---|---|
| TAMPON | NUMBER OF PANELISTS |
| M | 6 (18.2%) |
| R | 10 (30.3%) |
| NO PREFERENCE | 17 (51.5%) |

**TABLE VII**

| **SPECIFIC PREFERENCE** | | | | |
|---|---|---|---|---|
| | Number of Panelists for: | | | |
| SPECIFIC PREFERENCE | TAMPON R | TAMPON M | NO PREFERENCE | NO ANSWER |
| Protection from leakage | 7 (21.2%) | 5 (15.2%) | 21 (63.6%) | 0 |
| Ease of applicator insertion | 5 (15.2%) | 6 (18.2%) | 22 (66.7%) | 0 |
| Comfort in insertion | 5 (15.2%) | 5 (15.2%) | 23 (69.7%) | 0 |
| Ease of pushing tampon out of applicator | 1 (3.0%) | 5 (15.2%) | 27 (81.8%) | 0 |
| Ease of applicator withdrawal | 2 (6.1%) | 4 (12.1%) | 27 (81.8%) | 0 |
| Ease of Tampon Withdrawal | 2 (6.3%) | 4 (12.5%) | 26 (81.3%) | 1 |

**TABLE VIII**

| **OVERALL PERFORMANCE ON SCALE OF 1 TO 5 (1 = POOR; 5 = EXCELLENT)** | | |
|---|---|---|
| PERFORMANCE | TAMPON "M" | TAMPON "R" |
| Excellent | 5 (15.2%) | 5 (15.2%) |
| Very good | 11 (33.3%) | 10 (30.3%) |
| Good | 7 (21.2%) | 6 (18.2%) |
| Fair | 6 (18.2%) | 7 (21.2%) |
| Poor | 4 (12.1%) | 5 (15.2%) |
| **Mean** | **3.21** | **3.09** |

From this study it was observed that women detect no significant difference between the applicator of the invention and the commercially available polyethylene applicator. In view of the results of Example 3, the results of this study are quite surprising in demonstrating the effectiveness of the simple wax coating. In Example 3 the uncoated PVOH applicator was quite unfavorably received by tampon users. However, as shown by this Example, the coating in accordance with the invention allows the PVOH applicator to have statistically essentially the same degree of acceptance by tampon users as the currently available, non-dissolvable commercial polyethylene applicator.

In sum, 51.5% of the panelists expressed no overall preference for Tampon R over Tampon M, and 18.2% of the panelists had an overall preference for Tampon M over Tampon R. The mean overall performance of Tampon R was 3.09 whereas the mean overall performance of Tampon M was 3.21. As to specific preferences for Tampon R over Tampon M, a significant majority of the panelists (approximately 64 to 82%) had no preference. These results indicate that the panelists did not notice any difference between the PVOH-wax coated applicator of the invention and the polyethylene applicator. The present invention can therefore replace the presently used polyethylene applicator in terms of customer use and acceptance.

Having thus described in detail preferred embodiments of the present invention, it is to be understood that the invention defined by the appended claims is not to be limited by particular details set forth in the above description as many as apparent variations thereof are possible without departing from the spirit or scope of the present invention in its broader aspects.

## Claims

1. A tampon applicator comprising at least one elongated tubular member having an exterior surface; and a coating on the exterior surface; said tubular member comprising a water-soluble polymer; and said coating comprising a water-insoluble polymeric material selected from the group consisting of wax, hydrogenated vegetable oil, and food grade shellac.

2. The tampon applicator of claim 1 wherein the water-soluble polymer is selected from the group consisting of thermoplastic starch, polyvinyl alcohol, poly(alkylene oxide), hydroxyethyl cellulose, hydroxypropyl cellulose, polyvinyl pyrrolidone, polyacrylamide, polyacrylic acid, and polyvinyl methyl ether-co maleic anhydride.

3. The tampon applicator of claim 1 wherein the water-insoluble polymeric material is a wax.

4. The tampon applicator of claim 3 wherein the wax is selected from the group consisting of natural wax and synthetic wax.

5. The tampon applicator of claim 4 wherein the wax is a natural wax selected from the group consisting of animal wax, vegetable wax and mineral wax.

6. The tampon applicator of claim 5 wherein the wax is a vegetable wax selected from the group consisting of carnauba wax, candelilla wax, bayberry wax, and sugar cane wax.

7. The tampon applicator of claim 5 wherein the wax is a mineral wax selected from the group consisting of fossil or earth wax and petroleum wax.

8. The tampon applicator of claim 7 wherein the wax is selected from the group consisting of ozokerite wax, ceresin wax, montan wax, paraffin wax, microcrystalline wax and petrolatum wax.

9. The tampon applicator of claim 2 wherein the water-soluble polymer is polyvinyl alcohol or thermoplastic starch and the water-insoluble polymeric material is a wax.

10. The tampon applicator of claim 9 wherein the water-soluble polymer is polyvinyl alcohol and the wax is paraffin wax or ozokerite wax.

11. The tampon applicator of claim 1 wherein the coating has a thickness of about 0.1 to about 1.0 mils.

12. The tampon applicator of claim 11 wherein the coating has a thickness of about 0.5 mils.

13. A method for producing a water-resistant, degradable tampon applicator comprising at least one elongated tubular member, said method comprising:
forming said tubular member from a water-soluble polymer, and
coating an exterior surface of said tubular member with a water-insoluble polymeric material by tumbling the tubular member in the presence of the water-insoluble polymeric material at a temperature above the melting point of the water-insoluble polymeric material and below the melting temperature of the water-soluble polymer.

14. The method of claim 13 wherein the forming of the tubular member is by injection-molding.

15. The method of claims 13 or 14 wherein the tumbling is at a temperature below the softening point of the water-soluble polymeric material.

16. The method of claims 13 or 14 wherein the tumbling is at a temperature of about 10° to 20°F above the melting point of the water-insoluble polymeric material.

17. The method of any of claims 13 to 16 wherein the tumbling is performed within a vessel having an interior surface and the coating is performed by introducing a sufficient quantity of water-insoluble polymeric material into the vessel to coat the interior surface thereof, and then adding the formed tubular member to the vessel.

18. The method of any of claims 13 to 17 wherein the water-insoluble polymeric material is selected from the group consisting of wax, and hydrogenated vegetable oil.

19. The method of any of claims 13 to 18 wherein the water-soluble polymer is selected from the group consisting of thermoplastic starch, polyvinyl alcohol, poly(alkylene oxide), hydroxyethyl cellulose, hydroxypropyl cellulose, polyvinyl pyrrolidone, polyacrylamide, polyacrylic acid, and polyvinyl methyl ether-co maleic anhydride.

20. The method of any of claims 13 to 19 wherein the water-soluble polymer is polyvinyl alcohol and the water-insoluble polymeric material is a wax.
